# FASCICULE DE BREVET EUROPEEN

(11) **EP 0 578 535 B1**
(45) Date de publication et mention de la délivrance du brevet: **21.08.1996**
(21) Numéro de dépôt: 93401666.8
(22) Date de dépôt: 29.06.1993
(51) Int. Cl.: C07K 5/10, A61K 38/07

(54) **Nouveaux dérivés peptidiques actifs dans les processus d'adhesion cellulaire, leur procédé de préparation et les compositions pharmaceutiques qui les renferment**
Neue Peptidderivate im Prozess von Zelladhäsion wirksam, Verfahren zur deren Herstellung und pharmazeutische Zusammensetzungen die sie enthalten
New peptide derivates active in the process of cell adhesion, a method to prepare them end pharmaceutical compositions containing them

(30) Priorité: 30.06.1992 FR 9208004
(43) Date de publication de la demande: 12.01.1994
(73) Titulaire: ADIR ET COMPAGNIE, F-92415 Courbevoie Cédex (FR)
(72) Inventeur: Fauchere, Jean-Luc, F-92210 Saint-Cloud (FR); Morris, Angela Dawn, F-78220 Viroflay (FR); Thurieau, Christophe, F-75016 Paris (FR); Verbeuren, Tony, F-78540 Vernouillet (FR); Simonet, Serge, F-78700 Conflans-St-Honorine (FR)

(56) Documents cités:
- EP-A- 0 275 748
- EP-A- 0 410 767

## Description

La présente invention concerne de nouveaux dérivés peptidiques, leur procédé de préparation et les compositions pharmaceutiques qui les contiennent. Les composés de la présente invention trouvent une utilisation pharmacologique intéressante en tant qu'anti-agrégants plaquettaires.

On sait que la prévention de la thrombose et de l'athérosclérose est conditionnée par le contrôle et la régulation de l'agrégation plaquettaire. La formation d'un thrombus ou caillot de sang est liée à la présence de fibrinogène qui, par son interaction avec un récepteur spécifique, en provoquant l'adhésion cellulaire, participe à l'agrégation plaquettaire. Le mécanisme est général pour tous les inducteurs et, de fait, il est particulièrement intéressant de chercher à réguler ou inhiber l'agrégation dépendante du fibrinogène.

Des dérivés peptidiques possédant des propriétés anti-agrégantes sont déjà connus. On peut citer à titre d'exemple :
- le brevet EP-A-319506, où l'on trouve l'enchaînement peptidique :
   Arg-Gly-Asp-O-méthylthyrosine amide,
- le brevet US-A-4578079 qui décrit des peptides de la forme :
   -Arg-Gly-Asp-Thr-, -Arg-Gly-Asp-Cys- ou -Arg-Gly-Asp-Ser-, et
- le brevet EP-A-220957 faisant apparaître des peptides de la forme :
   -Lys-Arg-Gly-Asp- ou -Arg-Arg-Gly-Asp -.

Les brevets EP-A-275748 et EP-A-341915 proposent des tétrapeptides de la forme X₁-Arg-Gly-Asp-Trp-X₂ dans lesquels X₁ et X₂ peuvent être des résidus d'acides aminés naturels.

La présente invention concerne des dérivés peptidiques présentant une originalité par rapport aux composés décrits dans l'art antérieur. En effet, l'intensité des propriétés pharmacologiques des produits de l'invention a été optimisée en modifiant la longueur et la nature du substituant N-terminal du tétrapeptide.

Plus particulièrement, l'invention a pour objet de nouveaux dérivés peptidiques répondant à la formule générale (I) :

R₁ - A - Gly - Asp - Trp - R₂ (I)

dans laquelle :
- A représente un résidu arginine (Arg) ou lysine ( Lys),
- R₁ représente un résidu amino-acide linéaire, ramifié ou cyclique dont le groupement amine ne se trouve pas en position α du groupement carbonyle,
- R₂ représente le groupe -NH₂ ou -OH,
leurs stéréoisomères ainsi que leurs sels d'addition à un acide ou à une base pharmaceutiquement acceptable,
étant entendu que chaque amino-acide de la séquence peptidique est optiquement pur et peut être de configuration D ou L.

Les résidus acides aminés linéaires, ramifiés ou cycliques, dont le groupement amine n'est pas en position α, sont des résidus d'amino-acides choisis préférentiellement et de manière non limitative parmi l'acide 3-aminopropanoïque, 3-amino-2-méthylpropanoïque, 4-aminobutanoïque, 5-aminopentanoïque, 6-aminohexanoïque, 7-aminoheptanoïque, pyrrolidine-3-carboxylique, pipéridine-4-carboxylique, pyridine-4-carboxylique, quinoléine-4-carboxylique.

L'invention s'étend aussi au procédé de préparation des composés de formule (I) qui peuvent être obtenus par différentes méthodes telles que la synthèse séquentielle sur phase solide, la synthèse de fragments et leur couplage en solution, la synthèse enzymatique, la synthèse génétique par clonage et expression des gènes dans des bactéries transformées ou par des combinaisons diverses de ces techniques.

Les peptides de la présente invention sont généralement obtenus par synthèse sur phase solide selon la méthode décrite par B.W. ERICKSON et R.B. MERRIFIELD ("The proteins", solid-phase peptide synthesis, 3rd edition, 257-527, 1976).

Plus spécifiquement, le procédé de préparation des composés de l'invention suit la méthode de synthèse et de couplage des fragments en phase solide.

La phase solide peut-être l'une des résines polymériques utilisées conventionnellement, telle que, par exemple, un polystyrène réticulé par du divinylbenzène (1 à 2 %) sur lequel un bras d'attachement, par exemple le p-alkoxybenzylalcool ou la benzhydrylamine, est fixé, auquel le peptide est attaché au cours des couplages d'acides aminés, et qui permet, en fin de synthèse, la libération d'une fonction C-terminale acide, amide, alcool ou autre.

La liste des abréviations utilisées dans le procédé de synthèse suivant est donnée en annexe.

Le procédé de préparation des produits de l'invention est caractérisé en ce que chaque étape de la synthèse est composée de deux opérations, à savoir :
1) déprotection du substrat à l'aide d'une base appropriée comme la pipéridine, par exemple,
2) couplage d'un acide aminé protégé en présence d'un réactif de couplage classique de la synthèse peptidique, comme par exemple le couple DCC/HOBT, TBTU/DIEA, BOP/DIEA ou le DPPA,
les réactifs et substrats obtenus pour chaque étape étant :

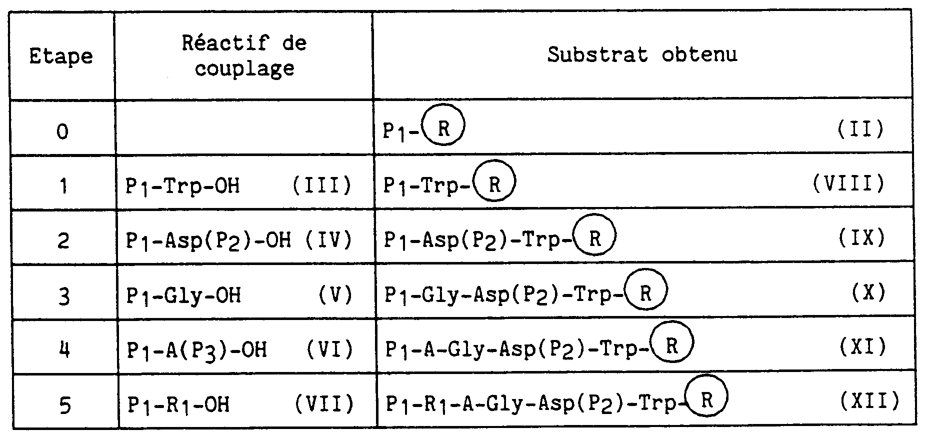

composés de formules (II) à (XII) dans lesquelles :
. R₁ et A sont tels que définis précédemment
. Ⓡ représente le support résine,
. P₁ est un groupement protecteur tel que Fmoc ou Boc par exemple,
. P₂ est un groupement protecteur de l'acide aspartique, comme par exemple, OtBu ou Bzl,
. P₃ est un groupement protecteur, pour l'arginine par exemple Pmc, ou pour la lysine par exemple Boc,
composés de formule (XII) qui sont ensuite libérés du support résine et des protecteurs d'acides aminés par un mélange de TFA, éthane-1,2-dithiol, anisole et éventuellement DCM, le groupement protecteur de l'acide aspartique, lorsque celui-ci est Bzl, étant éliminé par traitement par l'hydrogénocarbonate d'ammonium et palladium dans le méthanol,
pour conduire finalement aux peptides bruts de formule (I), que l'on purifie par une technique classique de purification et que l'on transforme, le cas échéant, en leurs sels d'addition à un acide ou à une base pharmaceutiquement acceptable.

Les composés de la présente invention sont doués de propriétés anti-agrégantes et peuvent être utilisés dans les cas de thromboembolisme par dissolution des caillots de sang ou en tant qu'agents préventifs de l'extension du processus thrombotique en les utilisant comme anticoagulants d'action directe et rapide, ou dans toute autre situation pathologique mettant en jeu des processus d'adhésion cellulaire.

Ils s'avèreront donc efficaces dans le traitement des thromboses, de l'embolie pulmonaire, de l'embolie artérielle des extrémités, de l'athérosclérose et des manifestations thrombolytiques, mais aussi de l'infarctus du myocarde et dans le traitement des re-sténoses après angioplasties, ainsi que pour maintenir l'homéostasie sanguine en particulier dans la circulation extracorporelle.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques de l'invention, on pourra citer plus particulièrement celles qui conviennent pour l'administration par voie orale, parentérale, nasale, les comprimés simples, dragéifiés ou sublingaux, les sachets, paquets, gélules, glossettes, tablettes, suppositoires, crèmes, pommades, gels dermiques, aérosols.

La posologie varie selon l'âge et le poids du patient, la nature et la sévérité de l'affection ainsi que la voie d'administration. Celle-ci peut être orale, nasale, rectale ou parentérale. D'une manière générale, elle s'échelonne entre 0,2 et 100 mg pour un traitement en une ou plusieurs prises par 24 heures.

ANNEXE

Abréviations utilisées
dans la présente invention

-------------------------

Boc : tert-butyloxycarbonyl.
BOP : benzoxytriazol-1-oxytris diméthylaminophosphonium hexafluorophos-
phate.
Bzl : benzyl.
DCC : 1,3-dicyclohexylcarbodiimide.
DCM : dichlorométhane.
DIEA : diisopropyléthylamine.
DMF : diméthylformamide
DPPA : diphénylphosphorylazide.
Fmoc : 9-fluorénylméthylcarbonyl.
HOBT : 1 - hydroxybenzotriazole.
Pmc : 2,2,5,7,8,-pentaméthylchroman-6-sulfonyl.
OtBu : β-tert-butyl ester.
TFA : acide trifluoroacétique.

Les exemples suivants illustrent l'invention et ne la limitent en aucune façon.

### Exemple 1 :

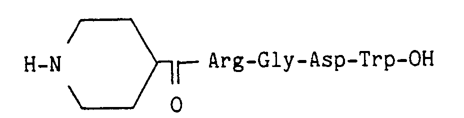

2,0 g de Fmoc-Trp, fixé sur une résine ester par l'intermédiaire d'un groupement p-alkoxybenzylalcool est placé sous agitation avec 30 ml d'une solution à 20 % en volume de pipéridine dans le diméthyl formamide pendant 30 minutes, afin de libérer le tryptophane de son groupement protecteur. Le mélange est filtré et la rés lavée avec les solvants DMF et DCM. Le fragment déprotégé est soumis à 1,81 g de Fmoc-Asp(OtBu)-OH dans 30 ml de DMF en présence de 997 mg de DCC et 743 mg de HOBT pendant 3 heures. Après filtration et lavage par DMF, alcool isopropylique et DCM, le milieu est traité comme précédemment par une solution de pipéridine à 20 % dans le DMF. Ce substrat est ensuite traité de la même manière avec 1,308 g de Fmoc-Gly-OH puis 2,916 g de Fmoc-Arg(Pmc)-OH et finalement avec 1,492 g de Fmoc-(acide iso-nipécotique) (Synthon préparé selon la procédure décrite par Carpino et Han (J. Org. Chem., 1972, 37, 3404)).
Le peptide est alors détaché du support résine et libéré des groupements protecteurs par traitement avec un mélange de 16 ml d'acide trifluroroacétique, 2 ml d'anisole, 2 ml d'éthanedithiol et 4 ml de DCM pendant deux heures à température ambiante. Après filtration et concentration du filtrat, le résidu est trituré dans du diéthyléther, puis filtré pour donner le peptide brut attendu. Le produit brut est purifié par chromatographie liquide à phase inverse (colonne c 18, gradient 10-35 % de CH₃CN en 25 minutes) pour conduire après lyophilisation au ditrifluoro acétate correspondant.
- Spectre de masse: (FAB) : [M+H]⁺ : m/z = 644
(masse moléculaire : 643).

Les composés des exemples ci-dessous ont été préparés selon le même mode opératoire que celui décrit dans l'exemple 1 avec les modifications suivantes :
- lorsque l'arginine est remplacée par la lysine le réactif utilisé est Fmoc-Lys(Boc)-OH au lieu de Fmoc-Arg(Pmc)-OH.
- La terminaison "amide" du tryptophane est obtenue en utilisant une résine du type 2,4-diméthoxy-4'-(carboxyméthyloxy)-benzhydrylamine qui libère le peptide amide par traitement par l'acide trifluoroacétique en fin de synthèse.
- Le Fmoc(acide iso-nipécotique) est remplacé par le résidu aminé correspondant, toujours protégé par Fmoc.

### Exemple 2 :

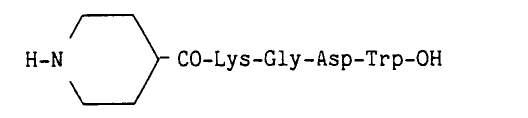

### Exemple 3 :

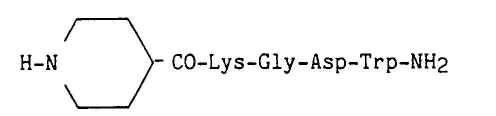

- Spectre de masse :: (FAB) : [M+H]⁺ : m/z = 615
(masse moléculaire : 614,7).

### Exemple 4 :

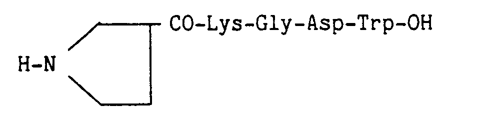

### Exemple 5 :

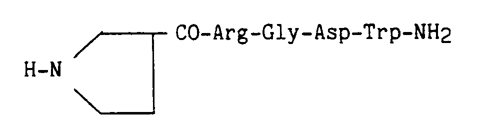

### Exemple 6 :

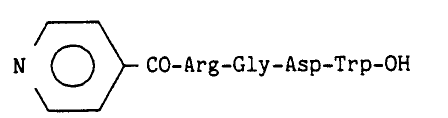

- Spectre de masse :: (FAB) : [M+H]⁺ : m/z = 638
(masse moléculaire : 637,6).

### Exemple 7 :

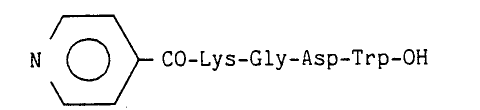

- Spectre de masse :: (FAB) : [M+H]⁺ : m/z = 610
(masse moléculaire : 609,6).

### Exemple 8 :

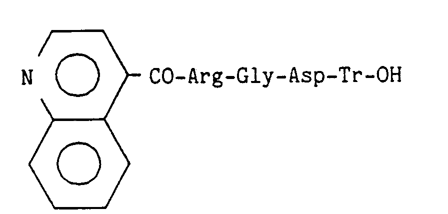

- Spectre de masse :: (FAB) : [M+H]^{+ :} m/z = 688
(masse moléculaire : 687,7).

### Exemple 9 :

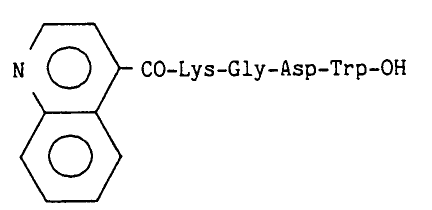

- Spectre de masse :: (FAB) : [M+H]^{+ :} m/z = 660
(masse moléculaire : 659,7).

### Exemple 10:

H₂N-(CH₂)₂-CO-Lys-Gly-Asp-Trp-OH

### Exemple 11 :

H₂N-(CH₂)₃-CO-Arg-Gly-Asp-Trp-OH
- Spectre de masse :: (FAB) : [M+H]⁺ : m/z = 618
(masse moléculaire : 617).

### Exemple 12:

H₂N-(CH₂)₃-CO-Lys-Gly-Asp-Trp-OH
- Spectre de masse :: (FAB) : [M+H]⁺ : m/z = 590
(masse moléculaire : 589).

### Exemple 13 :

H₂N-(CH₂)₄-CO-Arg-Gly-Asp-Trp-OH
- Spectre de masse :: (FAB) : [M+H]⁺ : m/z = 632
(masse moléculaire : 631).

### Exemple 14 :

H₂N-(CH₂)₅-CO-Arg-Gly-Asp-Trp-OH
- Spectre de masse :: (FAB) : [M+H]⁺ : m/z = 646
(masse moléculaire : 645).

### Exemple 15 :

H₂N-(CH₂)₅-CO-Lys-Gly-Asp-Trp-OH

### Exemple 16 :

H₂N-(CH₂)₅-CO-Lys-Gly-Asp-Trp-NH₂
- Spectre de masse :: (FAB) : [M+H]⁺ : m/z = 617
(masse moléculaire : 616,7).

### Exemple 17 :

H₂N-(CH₂)₆-CO-Arg-Gly-Asp-Trp-OH
- Spectre de masse :: (FAB) : [M+H]⁺ : m/z = 660
(masse moléculaire : 659).

### Exemple 18 :

H₂N-(CH₂)₆-CO-Lys-Gly-Asp-Trp-NH₂

### Exemple 19 :

H₂N-(CH₂)₇-CO-Arg-Gly-Asp-Trp-NH₂

### Exemple 20 :

H₂N-CH₂-CH(CH₃)-CO-Arg-Gly-Asp-Trp-OH

### ETUDE PHARMACOLOGIQUE

### Exemple 21 : Agrégation plaquettaire

L'étude est réalisée sur des plaquettes de chien. Après anesthésie de l'animal par le sodium pentobarbital (30 mg/kg i.v.), le sang artériel est prélevé sur citrate de sodium (0,109 M) (11 vol. de citrate pour 9 vol. de sang). Le plasma riche en plaquettes (PRP) est obtenu après centrifugation (20 °C) à 200 g pendant 10 minutes. le nombre de plaquettes dans le PRP est en moyenne de 300 000 pl/mm³. Le PRP est conservé à la température de la pièce jusqu'au moment du test et est utilisé dans les 4 heures qui suivent le prélèvement.
L'effet anti-agrégant des produits est examiné sur des plaquettes activées avec l'adénosine diphosphate (ADP) (10µM). L'agrégation plaquettaire est réalisée à 37 °C dans des tubes en verre siliconé, à l'aide d'un agrégomètre (Coultronics). Le PRP est agité à 1000 rpm. Pour tester l'effet de l'antagoniste, le PRP est incubé avec la substance pendant 9 minutes sans agitation et 1 minute avec agitation. Ensuite l'ADP (10µM) est ajouté.

L'effet des antagonistes est mesuré et l'IC₅₀ est déterminée comme la concentration de l'antagoniste nécessaire pour produire 50 % d'inhibition des réponses agrégantes à l'ADP.
Les résultats sont reproduits dans le tableau I suivant :

**Tableau I**

| **Inhibition de l'agrégation plaquettaire due au fibrinogène** | |
|---|---|
| **N° Exemple** | **IC**_{**50**} **(µM)** |
| Exemple 1 | 0,23 |
| Exemple 11 | 2,1 |
| Exemple 12 | 2,3 |
| Exemple 13 | 2,4 |
| Exemple 14 | 0,79 |
| Exemple 17 | 6,3 |

| **Composés de référence** | |
|---|---|
| Acétyl-*cyclo*(Cys-Arg-Gly-Asp-Pen)NH₂ * | 8,1 |
| H-Arg-Gly-Asp-OH ** | 22,6 |

| | |
|---|---|
| * Produit décrit dans EP-A-341915 | |
| ** Produit décrit dans EP-A-275748. | |

### Exemple 22 : Thrombose expérimentale dans l'artère mésentérique du rat.

Les expériences ont été effectuées chez des rats mâles (250 à 400 g). Les animaux sont anesthésiés avec le pentobarbital (50 mg/kg i.p.). Un cathéter est installé dans la veine jugulaire afin de permettre des injections i.v.

La préparation de l'artère mésentérique et l'induction du thrombose sont effectués selon la méthode décrite par Bourgain et al. Adv. Exp. Med. Biol., 180, 635-649, (1984). Une incision longitudinale est faite dans l'abdomen et une partie de l'intestin est exposée. A l'aide d'une loupe binoculaire, une branche de l'artère mésentérique d'un diamètre de 200 à 500 µm est préparée. La veine voisine est écartée et les tissus adipeux retirés ; un segment d'artère de 2 mm est ainsi préparé. L'animal est alors installé sous microscope et le segment de l'artère mésentérique est superfusé avec de la solution physiologique (37 °C) à 5 ml/min. Le segment peut être observé pendant plusieurs heures. Les images sont enregistrées à l'aide d'une caméra vidéo et d'un magnétoscope.

Après installation et stabilisation de la préparation, une électrode en platine est placée de part et d'autre de la paroi de l'artériole à l'aide d'un micromanipulateur. Une lésion endothéliale est alors induite en applicant durant 60 secondes un courant électrique de 40 µA, courant inversé chaque 5 secondes. Cette lésion est faible et ne provoque aucun changement du débit sanguin ou du diamètre du vaisseau. Normalement, aucune formation de thrombus n'est observée à l'application du courant électrique.
La formation d'un caillot est ensuite induite en utilisant une superfusion avec de l'ADP (adénosine disphosphate) pendant 2 minutes. L'ADP est utilisé à une molarité finale de 300 µM. Le caillot se forme, puis disparaît après 2 à 5 minutes par embolisation. Le vaisseau reprend ensuite ces conditions normales ; il n'y a pas de formation spontanée de caillot. Toutes les 15 minutes, la superfusion avec l'ADP peut être répétée et les caillots qui se forment ont une taille comparable.

L'effet des produits sur la formation des thrombus a été testé en traitant les animaux par infusion veineuse durant 5 minutes. L'infusion démarre 1 minute avant la superfusion avec l'ADP.

Les images enregistrées sont analysées, la taille des caillots est exprimée en valeurs arbitraires de surface. Les mesures sont effectuées à des temps déterminés pendant et après la superfusion d'ADP. La taille des caillots peut donc être comparée entre l'état contrôle et après infusion du produit.

Les résultats sont produits dans le tableau II suivant :

**Tableau II**

| **Pourcentage d'inhibition de formation d'un caillot de sang dans l'artère mésentérique du rat** | | | |
|---|---|---|---|
| **COMPOSES** | **% D'INHIBITION** | | |
| | **Infusion pendant 5 min** | | |
| | 2 mg/kg/min | 5 mg/kg/min | 10 mg/kg/min |
| Exemple 12 | 17 | 33 | 56 |
| H-Arg-Gly-Asp-Trp-OH (EP-A-275748) | - | - | 36 |

### Exemple 23 : Thromboembolisme chez la souris

Des souris mâles (CD-1) pesant 28-32 g sont utilisées pour ces expériences. Les animaux sont maintenus à 26-28 °C durant les 30 minutes qui précèdent l'expérimentation. La combinaison de collagène (150 µg/ml) et d'adrénaline (100 µmol/l) provoque la mort par la paralysie de la souris. Chaque souris reçoit 0,1 ml de cette association, ce qui donne une dose de 1,8 µg d'adrénaline et de 15 µg de collagène par souris. L'incidence de la mort ou de la paralysie est observée 15 minutes après l'injection intraveineuse (I.V) de la combinaison (adrénaline + collagène) dans la veine caudale. Les peptides sont injectés simultanément avec la combinaison des agonistes. Pour les tests portant sur la durée d'action, les souris sont prétraitées par les peptides par voie I.V. dans un volume de 0,1 ml.
Un examen histologique des poumons est réalisé afin de visualiser la présence de microthrombi dans les vaisseaux de la microcirculation, dus à la combinaison adrénaline-collagène. A la fin de l'expérimentation, les poumons sont rapidement enlevés, fixés dans le formol, puis inclus dans la paraffine. Des sections de 5 µm sont réalisées et colorées avec de l'hémaline/éosine. Les coupes sont examinées et la présence ou non de microthrombi est notée. Le test du X² (Fischer) est utilisé pour déterminer si il y a une différence significative dans les effets entre les souris ayant reçu les peptides et celles n'en ayant pas reçu.

Sur ce test les composés de l'invention sont actifs et protègent les souris contre les effets de collagène et de l'adrénaline ; p.e. le composé de l'exemple 11 protège la moitié des souris à une dose de 0,03 mg/souris et est 10 fois plus actif que la référence (RDGW = H-Arg-Gly-Asp-Trp-OH (EP-A-275743)) qui ne protège que la moitié des souris à une dose de 0,3 mg/souris. De plus, la durée d'action du RGDW à la dose de 1 mg est inférieure à 5 minutes tandis que les composés de l'invention, comme par exemple le composé de l'exemple 1, est supérieure à une heure. La formation de microthrombi dans le poumon est inhibée de façon plus importante avec les composés de l'invention qu'avec le RGDW. Par exemple, le composé de l'exemple 11 inhibe la formation des thrombi de façon significative à partir de 0,03 mg/souris. Le RGDW n'est actif qu'à partir de 1 mg/souris.

## Revendications

1. Dérivés peptidiques de formule générale (I) :
R₁ - A - Gly - Asp - Trp - R₂ (I)
dans laquelle :
- A représente un résidu arginine (Arg) ou lysine ( Lys),
- R₁ représente un résidu amino-acide linéaire, ramifié ou cyclique dont le groupement amine ne se trouve pas en position α du groupement carbonyle, et
- R₂ représente le groupe -NH₂ ou -OH,
leurs stéréoisomères ainsi que leurs sels d'addition à un acide ou à une base pharmaceutiquement acceptable,

2. Dérivés peptidiques selon la revendication 1 où R₁ est un résidu d'un amino-acide choisi parmi les acides 3-aminopropanoïque, 3-amino-2-méthylpropanoïque, 4-aminobutanoïque, 5-aminopentanoïque, 6-aminohexanoïque, 7-aminoheptanoïque, pyrrolidine 3-carboxylique, pipéridine-4-carboxylique, pyridine-4-carboxylique, et quinoléine-4-carboxylique,
leurs stéréoisomères ainsi que leurs sels d'addition à un acide ou à une base pharmaceutiquement acceptable.

3. Dérivés peptidiques selon la revendication 1 où A représente un résidu arginine (Arg),
leurs stéréoisomères ainsi que leurs sels d'addition à un acide ou à une base pharmaceutiquement acceptable.

4. Dérivés peptidiques selon la revendication 1 où A représente un résidu lysine (Lys),
leurs stéréoisomères ainsi que leurs sels d'addition à un acide ou à une base pharmaceutiquement acceptable.

5. Dérivés peptidiques selon la revendication 1 où R₂ représente le groupe -OH,
leurs stéréoisomères ainsi que leurs sels d'addition à un acide ou à une base pharmaceutiquement acceptable.

6. Dérivés peptidiques selon la revendication 1 où R₂ représente le groupe -NH₂,
leurs stéréoisomères ainsi que leurs sels d'addition à un acide ou à une base pharmaceutiquement acceptable.

7. Dérivés peptidiques selon la revendication 1 où R₁ représente le groupe -CO-(CH₂)₅-NH₂,
leurs stéréoisomères ainsi que leurs sels d'addition à un acide ou à une base pharmaceutiquement acceptable.

8. Dérivés peptidiques selon la revendication 1 où R₁ représente le groupe 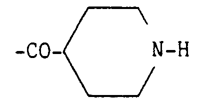 leurs stéréoisomères ainsi que leurs sels d'addition à un acide ou à une base pharmaceutiquement acceptable.

9. Dérivé peptidique selon la revendication 1 qui est le 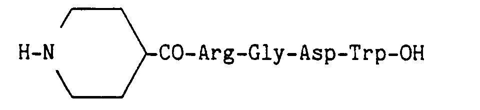 ses stéréoisomères ainsi que ses sels d'addition à un acide ou à une base pharmaceutiquement acceptable.

10. Dérivé peptidique selon la revendication 1 qui est le 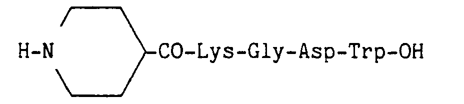 ses stéréoisomères ainsi que ses sels d'addition à un acide ou à une base pharmaceutiquement acceptable.

11. Procédé de préparation des dérivés peptidiques de formule (I) caractérisé en ce qu'ils peuvent être obtenus par synthèse séquentielle sur phase solide d'amino-acides protégés, synthèse enzymatique, synthèse génétique par clonage et expression des gènes dans des bactéries transformées ou par des combinaisons diverses de ces techniques,
dérivés de formule (I) que l'on purifie par une technique classique de purification et que l'on transforme, le cas échéant, en leurs sels d'addition à un acide ou à une base pharmaceutiquement acceptable.

12. Compositions pharmaceutiques contenant comme principe actif au moins un composé selon l'une quelconque des revendications 1 à 10, seul ou en combinaison avec un ou plusieurs excipients ou véhicules inertes, non toxiques, pharmaceutiquement acceptables.

13. Compositions pharmaceutiques selon la revendication 12 contenant au moins un principe actif selon l'une quelconque des revendications 1 à 10 utiles comme anti-agrégants dans le traitement des thromboses, de l'embolie pulmonaire, de l'embolie artérielle des extrémités, de l'infarctus du myocarde, de l'athérosclérose et des manifestations thromboemboliques, des re-sténoses après angioplasties, ainsi que pour maintenir l'homéostasie sanguine en particulier dans la circulation extracorporelle, et comme thérapie adjuvante au traitement thrombolytique.

## Claims

1. Peptide compounds of the general formula (I):
R₁ - A - Gly - Asp - Trp - R₂ (I)
in which:
- A represents an arginine (Arg) or lysine (Lys) residue,
- R₁ represents a linear, branched or cyclic amino acid residue, the amine grouping of which is not in the α-position relative to the carbonyl grouping, and
- R₂ represents the group -NH₂ or -OH,
their stereoisomers and also their addition salts with a pharmaceutically acceptable acid or base.

2. Peptide compounds according to claim 1 in which R₁ is a residue of an amino acid selected from 3-aminopropanoic acid, 3-amino-2-methylpropanoic acid, 4-aminobutanoic acid, 5-aminopentanoic acid, 6-aminohexanoic acid, 7-aminoheptanoic acid, 3-pyrrolidine carboxylic acid, 4-piperidinecarboxylic acid, 4-pyridinecarboxylic acid and 4-quinolinecarboxylic acid, their stereoisomers and also their addition salts with a pharmaceutically acceptable acid or base.

3. Peptide compounds according to claim 1 in which A represents an arginine residue (Arg), their stereoisomers and also their addition salts with a pharmaceutically acceptable acid or base.

4. Peptide compounds according to claim 1 in which A represents a lysine residue (Lys), their stereoisomers and also their addition salts with a pharmaceutically acceptable acid or base.

5. Peptide compounds according to claim 1 in which R₂ represents the group -OH, their stereoisomers and also their addition salts with a pharmaceutically acceptable acid or base.

6. Peptide compounds according to claim 1 in which R₂ represents the group -NH₂, their stereoisomers and also their addition salts with a pharmaceutically acceptable acid or base.

7. Peptide compounds according to claim 1 in which R₁ represents the group -CO-(CH₂)₅-NH₂, their stereoisomers and also their addition salts with a pharmaceutically acceptable acid or base.

8. Peptide compounds according to claim 1 in which R₁ represents the group 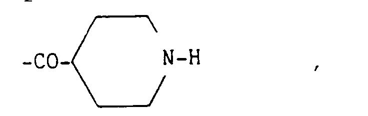 their stereoisomers and also their addition salts with a pharmaceutically acceptable acid or base.

9. Peptide compound according to claim 1 which is 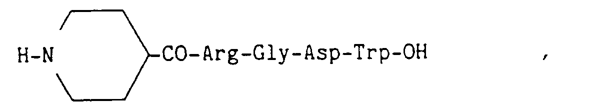 its stereoisomers and also its addition salts with a pharmaceutically acceptable acid or base.

10. Peptide compound according to claim 1 which is 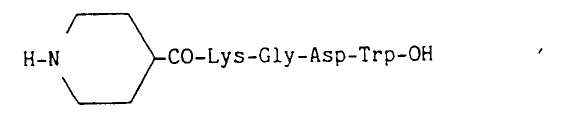 its stereoisomers and also its addition salts with a pharmaceutically acceptable acid or base.

11. Process for the preparation of the peptide compounds of formula (I), characterised in that they can be obtained by sequential solid-phase synthesis using protected amino acids, enzymatic synthesis, genetic synthesis by cloning and expression of genes in transformed bacteria or by various combinations of those techniques,
which compounds of formula (I) are purified by a conventional purification technique and which are converted, where appropriate, into their addition salts with a pharmaceutically acceptable acid or base.

12. Pharmaceutical compositions containing as active ingredient at least one compound according to any one of claims 1 to 10, alone or in combination with one or more pharmaceutically acceptable inert, non-toxic excipients or carriers.

13. Pharmaceutical compositions according to claim 12 containing at least one active ingredient according to any one of claims 1 to 10 for use as anti-aggregation agents in the treatment of thromboses, pulmonary embolism, arterial embolism of the extremities, myocardial infarct, atherosclerosis and thromboembolic symptoms, re-stenoses after angioplasty, and also in the maintenance of blood homoeostasis, especially in extracorporeal circulation, and as auxiliary therapy to thrombolytic treatment.

## Patentansprüche

1. Peptidderivate der allgemeinen Formel (I):
R₁ - A - Gly - Asp - Trp - R₂ (I)
in der:
- A einen Argininrest (Arg) oder einen Lysinrest (Lys),
- R₁ einen geradkettigen, verzweigten oder cyclischen Aminosäurerest, dessen Aminogruppe nicht in der α-Stellungzur Carbonylgruppe steht, und
- R₂ die Gruppe -NH₂ oder -OH bedeuten,
deren Stereoisomere sowie deren Additionssalze mit einer pharmazeutisch annehmbaren Säure oder Base.

2. Peptidderivate nach Anspruch 1, worin R₁ einen Rest einer Aminosäure ausgewählt aus 3-Aminopropansäure, 3-Amino-2-methylpropansäure, 4-Aminobutansäure, 5-Aminopentansäure. 6-Aminohexansäure, 7-Aminoheptansäure, Pyrrolidin-3-carbonsäure, Piperidin-4-carbonsäure, Pyridin-4-carbonsäure und Chinolin-4-carbonsäure, bedeutet,
deren Stereoisomere sowie deren Additionssalze mit einer pharmazeutisch annehmbaren Säure oder Base.

3. Peptidderivate nach Anspruch 1, worin A einen Argininrest (Arg) bedeutet, deren Stereoisomere sowie deren Additionssalze mit einer pharmazeutisch annehmbaren Säure oder Base.

4. Peptidderivate nach Anspruch 1, worin A einen Lysinrest (Lys) bedeutet, deren Stereoisomere sowie deren Additionssalze mit einer pharmazeutisch annehmbaren Säure oder Base.

5. Peptidderivate nach Anspruch 1, worin R₂ die Gruppe -OH bedeutet, deren Stereoisomere sowie deren Additionssalze mit einer pharmazeutisch annehmbaren Säure oder Base.

6. Peptidderivate nach Anspruch 1, worin R₂ die Gruppe -NH₂ bedeutet,
deren Stereoisomere sowie deren Additionssalze mit einer pharmazeutisch annehmbaren Säure oder Base.

7. Peptidderivate nach Anspruch 1, worin R₁ die Gruppe -CO-(CH₂)₅-NH₂ bedeutet,
deren Stereoisomere sowie deren Additionssalze mit einer pharmazeutisch annehmbaren Säure oder Base.

8. Peptidderivate nach Anspruch 1, worin R₁ die Gruppe 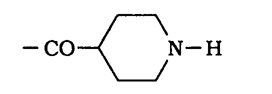 bedeutet, deren Stereoisomere sowie deren Additionssalze mit einer pharmazeutisch annehmbaren Säure oder Base.

9. Peptidderivat nach Anspruch 1, nämlich 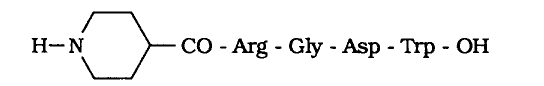 bedeutet, dessen Stereoisomere sowie dessen Additionssalze mit einer pharmazeutisch annehmbaren Säure oder Base.

10. Peptidderivat nach Anspruch 1, nämlich 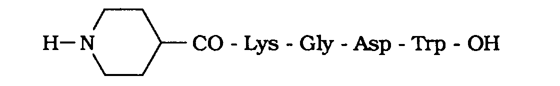 bedeutet, dessen Stereoisomere sowie dessen Additionssalze mit einer pharmazeutisch annehmbaren Säure oder Base.

11. Verfahren zur Herstellung der Peptidderivate der Formel (I) **dadurch gekennzeichnet**, daß sie durch sequentielle Festphasensynthese von geschützten Aminosäuren, enzymatische Synthese, genetische Synthese durch Klonierung und Expression der Gene in transformierten Bakterien oder durch verschiedene Kombinationen dieser Methoden erhältlich sind, welche Derivate der Formel (I) man mit einer klassischen Reinigungsmethode reinigt odergegebenenfalls in ihre Additionssalze mit einer pharmazeutisch annehmbaren Säure oder Base umwandelt.

12. Pharmazeutische Zubereitungen enthaltend als Wirkstoffmindestens eine Verbindung nach einem der Ansprüche 1 bis 10 allein oder in Kombination mit einem oder mehreren inerten, nichttoxischen, pharmazeutisch annehmbaren Trägermaterialien oder Bindemitteln.

13. Pharmazeutische Zubereitungen nach Anspruch 12, enthaltend mindestens einen Wirkstoff nach einem der Ansprüche 1 bis 10, die als Antiaggregationsmittel bei der Behandlung vonThrombosen, von Lungenembolien, von Arterienembolien in den Extremitäten, von Myokardinfarkten, der Atherosklerose und von thromboembolischen Manifestationen, Re-Stenosen nach Angioplastien sowie zur Aufrechterhaltung der Bluthämostasie, insbesondere in der extrakorporalen Zirkulation, und führ die Adjuvanstherapie bei der thrombolytischen Behandlung geeignet sind.
